# EUROPEAN PATENT APPLICATION

(11) **EP 3 858 424 A1**
(43) Date of publication of application: **04.08.2021**
(21) Application number: 20154366.7
(22) Date of filing: 29.01.2020
(51) Int. Cl.: A61N 1/05, A61M 25/00

(54) **TUBE ASSEMBLY AND MEDICAL PRODUCT COMPRISING SUCH A TUBE ASSEMBLY**

(71) Applicant: BIOTRONIK SE & Co. KG, 12359 Berlin (DE)
(72) Inventor: Trip, Erik, 090112 Singapur (SG); Kolberg, Gernot, 12161 Berlin (DE); Zedler, Stephan, 12309 Berlin (DE)
(74) Representative: Biotronik Corporate Services SE

(57) **Abstract**

The invention relates to a tube assembly having an inner tube segment (2) and an outer tube segment (3), wherein the outer tube segment (3) completely surrounds the inner tube segment (2) in at least a first central longitudinal portion (ZL) of the inner tube segment (2) along an outer circumference of the inner tube segment (2), wherein the outer tube segment (3) comprises a first polymer, which has a first melting range, and the inner tube segment (2) comprises a second polymer, which has a second melting range or a decomposition temperature, wherein a mean value of the second melting range or, if the second polymer does not have a melting range, but instead only a decomposition temperature, the decomposition temperature is at least 50°C higher than a mean value of the first melting range. The tube assembly (1) is characterised in that it comprises a first terminal longitudinal portion (TL1), in which the outer tube segment (3) is arranged, but the inner tube segment (2) is not.

## Description

The present invention relates to a tube assembly according to the preamble of claim 1, a medical product comprising such a tube assembly according to the preamble of claim 10, and a method for producing such a tube assembly according to the preamble of claim 13.

Tube assemblies, for example tubes inserted one inside the other, are already used in numerous technical fields. They are also used in the medical sector in order to combine the advantageous material properties of different materials with one another.

For example, insulation tubes for electrodes of cardiac pacemakers are known from US 2009/0069877 A1 and US 2009/0208635 A1. These insulation tubes may be made on their inner side for example of a silicone and on the outer side of a polyurethane.

Polyurethanes have attractive properties for implants such as electrodes of this kind. They are wear-resistant and have good anti-friction properties. They may thus be used with very thin wall thicknesses and yet still have good insulation properties. Polyurethanes, like other thermoplastics, may also be easily connected by welding and may thus be assembled economically and very effectively.

One problem with polyurethanes is that they have a lower biostability as compared to silicones. For example, they are more susceptible to degradation by metal ion oxidation (in particular in the case of direct metal contact) and by environment stress cracking (ESC).

By contrast, silicone is extremely biostable, but has a lower wear resistance as compared to polyurethanes and therefore must be used fundamentally with greater wall thicknesses. It is typically possible to connect a component made of silicone to another component only by means of an adhesive, which within the medical sector must also be biocompatible, stable under moist conditions, and additionally durable under all circumstances. Such adhesives are typically awkward to handle and generally require a long resting and curing time.

By way of the products known on the market, the advantageous properties of silicones on the one hand and polyurethanes on the other hand may be combined with one another. However, at the same time, the disadvantageous properties of both materials are also combined and have to be taken into consideration in the further processing of the corresponding components.

For the production of composite materials of this kind, it is known to coat a silicone tube with a thermoplastic material by inserting two independent tubes one inside the other. These tubes may lie loosely one over the other or may sit tightly one on the other, or may also be connected to one another (in part).

A further possibility for producing a composite material of this kind lies in extruding a thermoplastic over a silicone tube.

In addition, it is possible to apply a thermoplastic tube to a silicone tube by dipping the silicone tube into a dissolved thermoplastic.

Composite tubes of this kind are known for example from US 5,358,516 A, US 2004/0098095 A1 and US 2004/0267197 A1.

The insertion of two or more tubes one inside the other has a number of disadvantages. Firstly, the process is relatively complex. Two tubes have to be manufactured with a very high degree of accuracy. The tubes are then slid one inside the other in a tube apparatus, which is typically very large. During this process, undesirable inclusions may become trapped between the two tubes. The accuracy of the fit between the two tubes ultimately determines the quality and the properties of the composite tube. A firmly adhering fit of the upper tube on the lower tube should be achieved - if at all - at most by a prior elastic expansion, swelling and/or by a subsequent shrinking of the upper tube. Consequently, a number of processes that are susceptible to faults are necessary in order to produce a tube assembly of this kind from a composite material.

If a thermoplastic material is extruded onto a silicone tube or is deposited from a solution onto a silicone tube, this in principle constitutes a good possibility for producing a stable and very accurately fitting tube assembly. A problem here is that such a tube assembly must be further processed as a silicone tube (that is to say in particular by an adhesive bonding to another component), since the ends of a tube assembly of this kind comprise both a silicone and a thermoplastic.

The object of the present invention is to provide a tube assembly that, as necessary, is resistant to wear and also biocompatible, and which may be further processed more easily than comparable tube assemblies from the prior art.

This object is achieved by a tube assembly having the features of claim 1. A tube assembly of this kind has an inner tube segment and an outer tube segment. Both tube segments extend along a direction of longitudinal extent of the tube assembly. This direction of longitudinal extent may be divided into longitudinal portions. In at least a first central longitudinal portion of the inner tube segment, the outer tube segment surrounds the inner tube segment completely, more specifically along an outer circumference of the inner tube segment. This means that the outer tube segment, in this first central longitudinal portion of the inner tube segment, lies completely around the inner tube segment on the outer side of the inner tube segment; the inner tube segment is embedded on its outer side in the outer tube segment. This first central longitudinal portion typically accounts for the greatest region of the longitudinal extent (extent in the direction of longitudinal extent) of the inner tube segment, for example 80 to 100% of the longitudinal extent of the inner tube segment, in particular 85% to 99%, in particular 90% to 95%.

The outer tube segment comprises a first polymer or is produced completely from the first polymer. This first polymer has a first melting range. The inner tube segment comprises a second polymer or is produced completely from the second polymer. The second polymer has a second melting range, or - if it is a polymer not capable of melting - a decomposition temperature. The term "polymer" shall be understood to mean a "polymeric plastic".

The first polymer is capable of melting more easily than the second polymer. Consequently, the outer tube segment may be converted more easily into a thermally deformable state or a molten state than the inner tube segment. Specifically, a mean value of the second melting range or - if the second polymer does not have a melting range, but only has a decomposition temperature - the decomposition temperature is at least 50°C higher than a mean value of the first melting range, in particular at least 75°C higher, in particular at least 100°C higher, in particular at least 125°C higher, in particular at least 150°C higher, in particular at least 170°C higher, in particular at least 200°C higher. The mean value of the second melting range or the decomposition temperature of the second polymer may be, for example, 50°C to 500°C, in particular 75°C to 450°C, in particular 100°C to 400°C, in particular 150°C to 350°C, in particular 200°C to 300°C higher than the mean value of the first melting range.

The melting range of the first polymer and/or the second polymer is preferably determined in accordance with DIN EN ISO 3146.

In accordance with the invention the tube assembly is characterised in that it has a first terminal longitudinal portion, in which there is arranged, of the two tube segments, only the outer tube segment, but not the inner tube segment. The term "terminal longitudinal portion" in this context refers to a portion of the longitudinal extent of the tube assembly that lies at one of the two ends of the tube assembly. Consequently, the tube assembly is formed in such a way that at least one end of the tube assembly comprises merely the first polymer, but not the second polymer. As explained, the first polymer is capable of melting more easily than the second polymer. The advantageous melting properties of the first polymer may thus be utilised for the further processing of the tube assembly, in particular for its connection to other components, whereas the advantageous properties of the second polymer or of the inner tube segment may still be utilised in the other longitudinal portions of the tube assembly. In this way, a wear-resistant, biocompatible, easily further-processible tube assembly may be produced.

Consequently, the tube assembly claimed in accordance with the invention combines the advantageous properties of various materials and yet, if the inner tube segment is produced for example from a silicone, does not require any additional adhesive in order to connect the entire tube assembly to another component. Rather, for a connection of this kind, the outer tube segment may be melted in the terminal region of the tube assembly by heating and then connected to another component. Consequently, in the case of the tube assembly claimed in accordance with the invention, merely a single additional manufacturing step (specifically the mounting of the outer tube segment on the inner tube segment, which for example may be achieved by an extrusion process) is necessary in order to produce a component that combines the advantages of two different polymer materials with one another. The solution claimed in accordance with the invention thus enables very favourable possibilities for producing a tube assembly. The production of the tube assembly also takes place at a relatively early manufacturing point of a more complex product, for example of a medical product, such that the tube assembly, in the event of a fault, does not have a high material value and may be easily replaced by a fault-free product.

In a variant, the first terminal longitudinal portion has a length (in the direction of longitudinal extent of the tube assembly) lying in the range of from 0.1 cm to 10 cm, in particular from 0.1 cm to 5 cm, in particular from 0.1 cm to 3 cm, in particular from 0.1 cm to 2 cm, in particular from 0.2 cm to 2 cm, in particular from 0.2 cm to 1 cm.

The inner tube segment and the outer tube segment may be connected to one another in principle in form-fitting fashion, in integrally bonded fashion and/or force-fitting fashion. Connection variants that comprise at least one form-fitting component and/or an integrally bonding component are considered to be more suitable in principle than connections that are produced merely by a force fit. If the outer tube segment is extruded over the inner tube segment, there is typically not a complete integral bond between the two tube segments.

This is because the outer tube segment may detach from the inner tube segment over time, even in the case of an extrusion of this kind of the outer tube segment over the inner tube segment. Typically, van der Waals forces are formed between the outer tube segment and the inner tube segment and are in principle more suitable for a durable connection of the outer tube segment to the inner tube segment than pure frictional forces between the two tube segments.

In a variant, at least a form-fitting connection, or exclusively a form-fitting connection is produced between the inner tube segment and the outer tube segment. In a further variant at least an integrally bonded connection, or exclusively an integrally bonded connection is produced between the outer tube segment and the inner tube segment.

In a variant, the tube assembly comprises a second terminal longitudinal portion, in which there is arranged the inner tube segment, but not the outer tube segment. In this case, the second terminal longitudinal portion is situated opposite the first terminal longitudinal portion of the tube assembly in the direction of longitudinal extent of the tube assembly (one tube assembly has precisely two terminal longitudinal portions).

In one variant the second terminal longitudinal portion has a length (in the direction of longitudinal extent of the tube assembly) that lies in particular in the range of from 0.1 cm to 10 cm, in particular from 0.1 cm to 5 cm, in particular from 0.1 cm to 3 cm, in particular from 0.1 cm to 2 cm, in particular from 0.2 cm to 2 cm, in particular from 0.2 cm to 1 cm. In one variant, the second terminal longitudinal portion is exactly the same size as the first terminal longitudinal portion.

The above-explained embodiment is particularly suitable for example if the tube assembly is to be used to produce a medical product which is to be connected only at one end to another component. If the tube assembly is to be used for example to produce a catheter, the proximal end of the tube assembly that is to be connected to a further component of the catheter may be formed as a first terminal longitudinal portion, that is to say as a longitudinal portion which may be easily melted and connected to a further component. It is then furthermore expedient to form a distal end region of the tube assembly in the form of the second terminal longitudinal portion, in order to thus achieve the most atraumatic design possible of this region of the catheter. This is because this second terminal longitudinal portion for example may then comprise only a particularly soft silicone, which in principle may not be easily connected to other materials (which is not even necessary, however, at a distal end of a catheter), but at the same time has properties that are very gentle on vessels.

In one variant the tube assembly has two terminal longitudinal portions, in which the outer tube segment is arranged, but the inner tube segment is not. This variant is expedient in particular if the tube assembly is to be used to produce a product in which the tube assembly is to be connected at its two ends to further components of the product. This is because such an embodiment of both terminal longitudinal portions ensures a particularly simple connection with utilisation of the advantageous connection properties of the first polymer of the outer tube portion. By contrast, the tube assembly, also in this embodiment, has the advantageous properties of the first polymer and of the second polymer (for example a high biocompatibility of an inner region and a high wear resistance of an outer region of the tube assembly) in its central longitudinal portion situated between the two terminal longitudinal portions.

In one variant the individual longitudinal portions of the tube assembly have different hardnesses, even though they are in essence made of the same polymer (with different degrees of hardness). For example, a core segment in a first terminal longitudinal portion of the tube assembly may have a first hardness, a core segment in a second terminal longitudinal portion of the tube assembly may have a second hardness, and the polymer used to extrude the outer tube segment may have a third hardness. The first hardness may be adapted for example to the hardness of the adjoining inner tube segment made of the second polymer. The second hardness for example may be higher than the first hardness. Lastly, the third hardness may be greater than the first hardness and the second hardness. A relatively hard polymer is then used to form the outer tube segment. During the extrusion it connects to the first core segment and the second core segment, which are produced from the same chemical material, but with a different hardness. A harder embodiment of the terminal longitudinal portion on one side of the inner tube segment thus results as compared to the terminal longitudinal portion on the other side of the inner tube segment.

Suitable hardnesses are, for example, for the first core segment a Shore hardness in a range of from 40A to 70A, in particular from 50A to 60A, for the second core segment a Shore hardness in a range of from 60A to 90A, in particular from 70A to 80A, and for the third hardness a Shore hardness in a range of from 45D to 80D, in particular from 50D to 75D, in particular from 55D to 70D. The Shore hardness is measured here preferably in accordance with DIN ISO 7619-1.

In order to produce a tube assembly having longitudinal portions of different hardness, a method is suitable in which a first core segment, the inner tube segment, and a second core segment are threaded successively onto a core wire. The individual segments are then fixed in such a way that they are no longer movable relative to one another. The first polymer may then be extruded over the arrangement formed of the first core segment, the inner tube segment and the second core segment, in order to form the outer tube segment. The first core segment and the second core segment typically likewise consist of the first polymer, wherein they have obtained another degree of hardness by way of a modification. Equally, an integrally bonded connection on the one hand between the extruded first polymer and the first core segment and on the other hand between the extruded first polymer and the second core segment is achieved as a result of the extrusion process. The two terminal longitudinal portions of the tube assembly, in the case of this production method, thus also consist merely of the first polymer or the outer tube segment, but not of the inner tube segment.

In a further variant it is provided that the tube assembly has at least one further central longitudinal portion, in which the outer tube segment is arranged, but not the inner tube segment. A further central longitudinal portion of this kind is characterised in that it is arranged between the two terminal longitudinal portions of the tube assembly and adjacently to at least one of the first central longitudinal portions of the tube assembly. If, in a further central region of this kind, only the outer tube segment is provided, such a further central longitudinal portion, during the production of a product from the tube assembly, may be provided for example in order to incorporate additional components there into the tube assembly. If the tube assembly is provided for example as insulation for an electrode, a ring electrode may be arranged in a further central longitudinal portion of this kind, and the functionality of said ring electrode may then not be disturbed by the inner tube segment (which is not provided in this central longitudinal portion).

In a variant it is envisaged that the inner tube segment is not only embedded on its outer side in the material of the outer tube segment, but is also embedded on its inner side in another material. For example, this may be the same material as the material of the outer tube segment, that is to say the first polymer or a material comprising the first polymer. The inner tube segment is then embedded in the form of a reinforcement element in the other material.

By contrast, in a further embodiment it is envisaged that an inner side of the inner tube segment defines or delimits an inner lumen of the tube assembly. This means that a fluid flowing through the inner lumen of the tube assembly comes into direct contact with the inner tube segment. In this way, it is particularly easily possible to utilise the advantageous material properties of the second polymer. For example, the inner lumen of the tube assembly may be particularly biocompatible if this is expedient for the subsequent purpose of the tube assembly.

In a variant the tube assembly is formed as a single-lumen tube assembly, that is to say has precisely one lumen. In another variant the tube assembly is formed at least in part with multiple lumens, that is to say has at least a plurality of lumens in some sections. In a variant, the tube assembly is formed fully as a multi-lumen tube assembly.

In a variant the tube assembly is formed in such a way that a lateral lumen of a terminally arranged tube segment is converted into a central lumen of the inner tube segment. This is expedient for example in the case of asymmetrically arranged electrode constructions of implantable cardioverters/defibrillators, in which a stylet lumen is guided laterally because there is no central lumen provided. In order to also be able to connect a stylet lumen of this kind to a head assembly, it must be centred with respect to the distal end of a multi-lumen tube of this kind. This may be achieved via the correspondingly arranged terminal tube segment.

In a variant the tube assembly has a mesh which is arranged between the inner tube segment and the outer tube segment. A mesh of this kind improves the introduction of force from one tube segment into the other tube segment and thus leads to an even closer connection of both tube parts to one another. A mesh of this kind may be made for example of a metal or of a polymer material. The polymeric plastic may be identical to the first polymer or the second polymer. In one embodiment the mesh comprises an aramid, for example Kevlar, or consists completely of an aramid, such as Kevlar.

In one embodiment the mesh is also arranged in the first terminal longitudinal portion of the tube assembly, in which merely the outer tube segment is provided, and not the inner tube segment. The mesh may then also be used to reinforce a connection of the outer tube segment to a further component to which the tube assembly is connected, in particular by melting the outer tubular segment in the first terminal longitudinal portion.

In a variant the first polymer is a thermoplastic. Furthermore, the second polymer in this variant is selected from the group consisting of thermoplastics, elastomers and thermosets. Thermosets are not capable of melting; they do not have a melting range, and instead merely have a decomposition temperature. A particularly suitable combination is a thermoplastic as first polymer and an elastomer as second polymer.

Particularly suitable thermoplastics of the first polymer and/or the second polymer are: polyurethanes (PU), polyester urethanes (PEU), polyether urethanes (PEEU), polycarbonate urethanes (PCU), silicone-based polycarbonate urethanes (PCU), polycarbonate-polyurea urethanes (PCHU), polydimethylsiloxane urethanes (PSU), polyisobutylene urethanes (PIU), polyisobutylene-based copolymers (PIC), polyether block amides (PEBA, for example PEBAX), thermoplastic polyimides (PI), fluorinated hydrocarbons, ethylene-tetrafluoroethylene copolymers (ETFE), polytetrafluorethylene (PTFE), polysulfone (PSU), polyethylene (PE), polypropylene (PP), polyamides (PA).

Particularly suitable thermoplastics for the second polymer are: polyimides (PI), fluorocarbons (PFC), ethyl ene-tetrafluoroethyl ene copolymers (ETFE), polytetrafluoroethylene (PTFE), tetrafluoroethylene (TFE), perfluoro(ethylene-propylene) (FEP), perfluoroalkoxy polymers (PFA), polyether ether ketones (PEEK).

Silicones are particularly suitable elastomers for the second polymer.

The inner tube segment is typically produced from one or more materials that have particularly suitable properties in respect of the intended use. For example, the one or more materials may have a particularly high biocompatibility and/or flexibility if the tube assembly is to be used for medical applications. By contrast, the outer tube segment is typically produced from one or more materials that for example provide the tube assembly with a high wear resistance and which may be easily welded or adhesively bonded to other components, or sealingly connected in some other way. The aforementioned materials offer a range of selection possibilities for choosing the first polymer and/or the second polymer in accordance with the particular specific requirements for the intended field of use of the tube assembly.

In one variant the first polymer is a polyurethane and the second polymer is a polytetrafluoroethylene (PTFE) or a silicone elastomer. In a further variant the first polymer is a polyurethane and the second polymer is a silicone elastomer. In this variant it is envisaged in particular that the outer tube segment consists completely of the first polymer and the inner tube segment consists completely of the second polymer. In this way, a tube assembly may be produced which is particularly well suited for medical applications and which may be used for example as an insulation tube for an electrode of a medical product or for the sheathing of a catheter.

One aspect of the present invention relates to a medical product which comprises a tube assembly according to the above explanations.

In one variant the medical product comprises an electrode which is implantable in a human or animal body. Alternatively or additionally the medical product comprises a catheter which is insertable into a human or animal body. The medical product may also be embodied in the form of an electrode of this kind or a catheter of this kind, that is to say might have no further components.

In one variant the medical product is a device for stimulating the human or animal heart or other organs, such as the stomach. Cardiac pacemakers, implantable cardioverters/defibrillators or devices for cardiac resynchronisation therapy are examples of apparatuses of this kind.

In a further embodiment the medical product is an apparatus for stimulating nerves. Neuro stimulators are examples of apparatuses of this kind.

In a further embodiment the medical product is a device for implanting a device for stimulating the human or animal heart. For example, this may be a catheter which is used for the implantation of a stimulation device. In a further embodiment the medical product is a device for implantation of a cardiac implant, for example an artificial heart valve. Examples of devices of this kind are insertion catheters, which are used within the scope of catheter-assisted subcutaneous heart valve replacement therapy.

One aspect of the present invention relates to a method for producing a tube assembly according to the above explanations. A method of this kind has the steps explained hereinafter.

An inner tube segment is firstly provided, which comprises a second polymer or consists completely of the second polymer. The second polymer has a second melting range, or a decomposition temperature if the second polymer is not capable of melting, but breaks down at an elevated temperature (such as a thermoset).

A first polymer is then extruded over the inner tube segment. An outer tube segment is thus formed around the inner tube segment. The first polymer has a first melting range.

A mean value of the second melting range or - if the second polymer does not have a melting range, but has only a decomposition temperature - the decomposition temperature is at least 50°C higher than the mean value of the first melting range.

The method is characterised in accordance with the invention in that the extrusion of the first polymer is performed not only in the areas in which the inner tube segment is arranged, but also at least in a first terminal longitudinal portion of the tube assembly to be produced, in which the inner tube segment is not arranged. The tube assembly thus has a first terminal longitudinal portion, in which the outer tube segment is arranged, but the inner tube segment is not. As explained above, in order to connect the tube assembly to a further component, the specific requirements of the first polymer with regard to the production of such a connection do not need to be taken into consideration. Rather, merely the material properties of the second polymer are decisive for the kind of connection to be produced.

In a variant, an additional tube assembly element is arranged ahead of or behind the inner tube segment in the direction of longitudinal extent or around the inner tube segment prior to the extrusion of the first polymer. The first polymer is then not only extruded over the inner tube segment, but also over the additional tube assembly element. Examples of a tube assembly element are an extrusion core with a mould contour which is to be moulded into an inner region of the outer tube segment formed by the first element, a mould part with an inner contour that is used for the subsequent connection of the tube assembly to a connection piece, and a mesh which is arranged around the inner tube segment.

In a further variant the following steps explained below are performed prior to the step of extrusion of the first polymer over the inner tube segment.

A core wire is firstly guided through an inner lumen of the inner tube segment. This core wire has, at a first end, a first core sleeve fixedly connected to the core wire. The core sleeve has an outer diameter which is greater than the diameter of the inner lumen of the inner tube segment. The core sleeve for example may have an outer diameter corresponding to the outer diameter of the inner tube segment. The core wire is guided through the inner lumen of the inner tube segment until the first core sleeve contacts a first end of the inner tube segment.

A second core sleeve is then slid onto the core wire until the second core sleeve contacts a second end of the inner tube segment. The inner tube segment is then in contact with each of its two ends with one of the two core sleeves. The second core sleeve is then fixed on the core wire so that it is no longer movable relative to the core wire. This may be achieved for example by means of a clamping screw, a thread on the core wire, a rubber ring, a bayonet closure, or a comparable connection. The second core sleeve has an outer diameter greater than the diameter of the inner lumen of the inner tube segment. For example, the second core sleeve may have an outer diameter corresponding to the outer diameter of the inner tube segment. It is consequently envisaged that the first core sleeve and the second core sleeve have structurally identical outer diameters.

In a next method step a mesh is applied to an outer side of the inner tube segment, the first core sleeve, and the second core sleeve. To this end, a ready-made mesh may be placed over the corresponding outer sides. In principle, it would also be conceivable to produce the mesh on the outer sides of the inner tube segment and the two core sleeves only once on site.

The first polymer is now extruded over the mesh and therefore also over the inner tube segment and the first core sleeve and the second core sleeve. However, materials of the first core sleeve and the second core sleeve are selected in such a way that there is not an integrally bonded connection between the extruded first polymer and the two core sleeves. Rather, the core sleeves are moved relative to the first polymer once the first polymer has cooled.

Following the extrusion step, the steps explained hereinafter are carried out.

Firstly, the fixing between the second core sleeve and the core wire is broken. The second core sleeve is removed from the core wire. This is performed in a first removal direction.

The core wire is now removed from the inner lumen of the inner tube segment together with the first core sleeve still fixedly connected to the core wire. This occurs in a second removal direction, which is opposite the first removal direction.

The inner tube segment, which is covered by a mesh and is extruded externally by the first polymer then remains, whereby an outer tube segment has been formed. Since the first polymer, however, was also extruded onto the mesh in the areas in which the core sleeves were arranged, the outer tube segment formed by the first polymer protrudes beyond the inner tube segment at both terminal longitudinal portions in the tube assembly. Consequently, the two terminal longitudinal portions of the formed tube assembly comprise merely the outer tube segment and the mesh, but not the inner tube segment.

By way of this method, a tube assembly that on the one hand is very stable, but on the other hand is also very flexible is provided, in which the inner tube segment and the outer tube segment are closely connected to one another with the aid of the intermediate mesh, and the risk of a detachment of the outer tube segment from the inner tube segment is significantly reduced. At the same time, the two terminal longitudinal portions of the formed tube assembly, in which merely the outer tube segment and the mesh are arranged, but not the inner tube segment, allow a particularly simple connection of the tube assembly to further components, in particular by a thermal welding method.

All variants and embodiments of the described tube assembly may be combined arbitrarily with one another and may be transferred in any arbitrary combination to the described medical product and the described method. Similarly, variants and embodiments of the described medical product may be combined arbitrarily with one another and transferred to the tube assembly and method. Lastly, all variants and embodiments of the described method may be combined with one another arbitrarily and transferred similarly to the tube assembly and the medical product.

Further details of aspects of the present invention will be explained in greater detail with reference to exemplary embodiments and corresponding figures, in which:
- Figure 1: shows a first exemplary embodiment of a tube assembly;
- Figure 2: shows a second exemplary embodiment of a tube assembly;
- Figure 3A: shows an exemplary embodiment of an extrusion core with inner tube segment fitted on it;
- Figure 3B: shows a third exemplary embodiment of a tube assembly which has been produced by means of the extrusion core in Figure 3A;
- Figure 4: shows a fourth exemplary embodiment of a tube assembly;
- Figure 5: shows a fifth exemplary embodiment of a tube assembly;
- Figure 6A: shows a first method step for producing a sixth exemplary embodiment of a tube assembly;
- Figure 6B: shows a second method step for producing the sixth exemplary embodiment of a tube assembly;
- Figure 6C: shows a third method step for producing the sixth exemplary embodiment of a tube assembly; and
- Figure 6D: shows a longitudinal section through the sixth exemplary embodiment of a tube assembly.

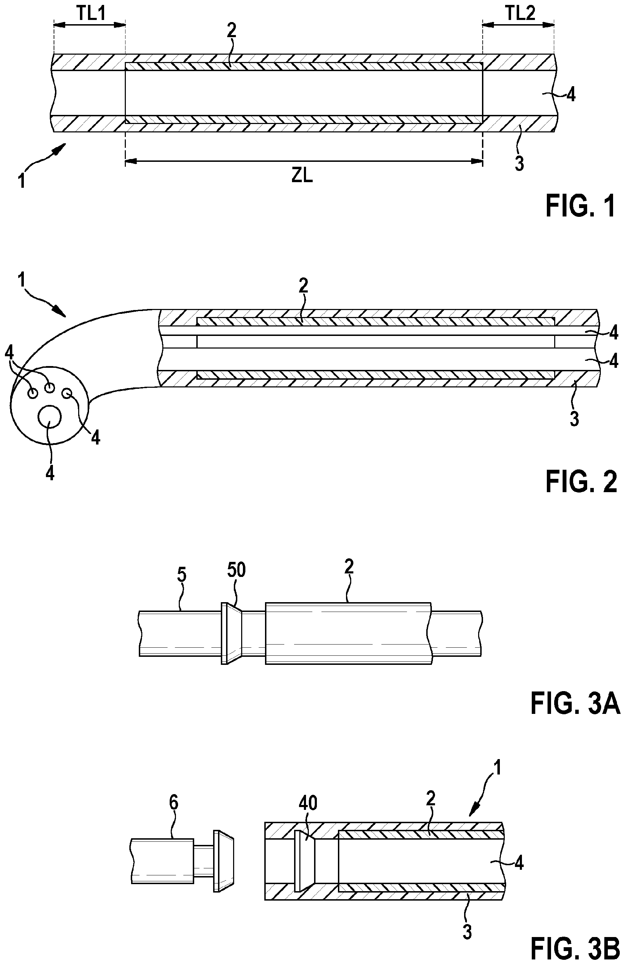

Figure 1 shows, in a longitudinal section, a first exemplary embodiment of a tube assembly 1, which comprises an inner tube segment 2 and an outer tube segment 3 extruded around this inner tube segment. The outer tube segment 3 is made of a thermoplastic, whereas the inner tube segment 2 is made of a silicone elastomer. A fluid that flows through an inner lumen 4 of the tube assembly comes into contact along a central longitudinal portion ZL only with the silicone elastomer of the inner tube segment 2, but not with the thermoplastic of the outer tube segment 3. By contrast, the thermoplastic of the outer tube segment 3 in the central longitudinal portion ZL of the tube assembly 1 provides a high wear resistance of the tube assembly 1 outwardly and thus ensures an extended service life of the tube assembly 1 in comparison to tube assemblies made only of a silicone elastomer.

In a first terminal longitudinal portion TL1 and a second terminal longitudinal portion TL2, the tube assembly 1 comprises only the outer tube segment 3, but not the inner tube segment 2. Consequently, the first terminal longitudinal portion TL1 and the second terminal longitudinal portion TL2 are made only of the thermoplastic of the outer tube segment 3, and therefore, in the event of connection of the tube assembly 1 to a further component, only the material properties of the thermoplastic of the outer tube segment 3 must be taken into consideration.

This thermoplastic of the outer tube segment 3 may melt typically at a relatively low temperature, such that a welded connection to another component is easily possible. By contrast, in conventional tube assemblies in which the inner tube segment protrudes as far as a terminal region of the tube assembly, the material properties of the material of the inner tube segment, for example a silicone elastomer, have to be taken into consideration. Silicone elastomers typically may not be connected to other components by welding, but only by adhesive bonding. For an adhesive bond of this kind, adhesives that are suitable for the particular application are necessary, for example biocompatible adhesives. These typically have long resting and curing times, and therefore a processing of tube assemblies of this kind known from the prior art is much more complex than a processing of the tube assembly 1 as in Figure 1.

Figure 2 shows a partially sectional view through a second exemplary embodiment of a tube assembly 1. In this figure and in all subsequent figures, similar elements are provided with the same reference signs as in the other figures. The tube assembly 1 in Figure 2 comprises an inner tube segment 2 and an outer tube segment 3. However, it is not only equipped with a single inner lumen 4, but with a total of four inner lumens 4. An embodiment of this kind of the tube assembly 1 may be achieved by extruding the thermoplastic of the outer tube segment 3 over the inner tube segment by means of four cores.

The terminal longitudinal portions TL1 and TL2 of the tube assembly 1 in Figure 2 are also produced exclusively from a thermoplastic that has been used for the extrusion of the outer tube segment 3 over the inner tube segment. The tube assembly 1 may thus also be fully connected to further components without adhesive steps.

Figure 3A shows an extrusion core 5 which has been guided through an inner lumen of an inner tube segment 2. The extrusion core 5 has a shaped contour 50, which corresponds, after the extrusion of a polymer onto the extrusion core 5, to an inner contour of the outer tube segment then extruded.

This is shown in Figure 3B, in which the inner tube segment 2 from Figure 3A has already been extruded externally by an outer tube segment 3. An inner contour 40 of the inner lumen 4 corresponding to the shaped contour 50 is formed on an inner side of the outer tube segment 3. This inner contour 40 is used to form a form-fitting connection to a head assembly 6, which may be introduced into the inner lumen 4 of the tube assembly 1 in order to connect the tube assembly 1 to a further component. The forming of a weld seam between the outer tube segment 3 of the tube assembly 1 and the head assembly 6 is additionally possible in order to produce a particularly tight connection between the tube assembly 1 and the head assembly 6.

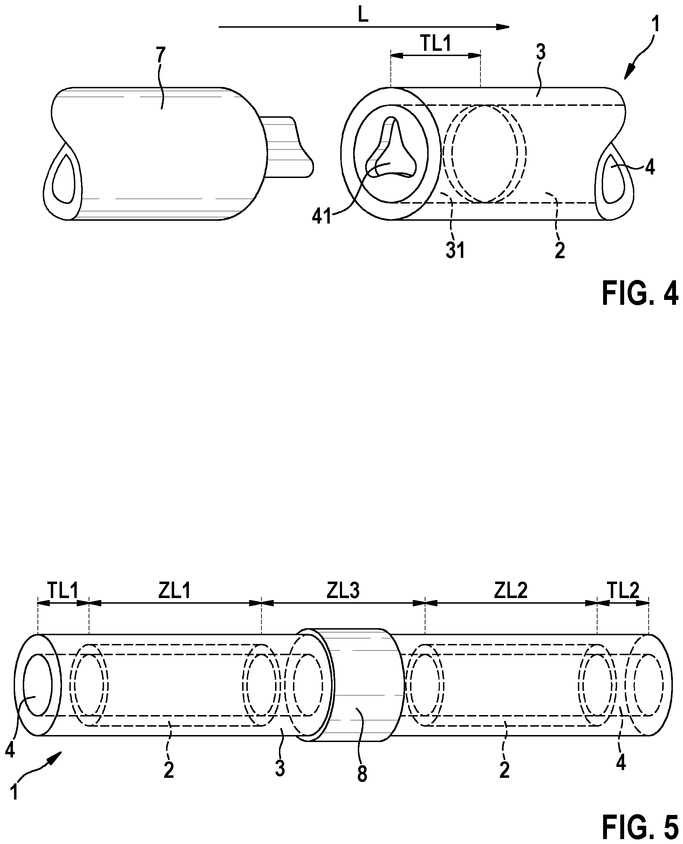

Figure 4 shows a tube assembly 1 with a multi-lumen inner tube segment 2, which has been drawn over three core wires in order to produce the tube assembly 1. A short shaped part 31, which serves as an additional tube assembly element and is made of the same polymer, in particular the same thermoplastic, as the outer tube segment 3, is arranged behind the inner tube segment 2 in the direction of longitudinal extent L. As the outer tube segment 3 is extruded over the inner tube segment 2 and the shaped part 31, an integrally bonded connection is thus formed between the shaped part 31 and the outer tube segment 3. The first terminal longitudinal portion TL1 of the tube assembly 1 is thus made merely of the first polymer of the outer tube segment 3. The shaped part 31 already has an inner contour 41, which is connected to the individual lumens 4 of the tube assembly 1 and enables a subsequent connection to a specifically designed connection piece 7.

Figure 5 shows a schematic depiction of a further exemplary embodiment of a tube assembly 1. Here, it is not only the thermoplastic of the outer tube segment 3 that is arranged in the first terminal longitudinal portion TL1 and in the second terminal longitudinal portion TL2. Rather, this tube assembly 1 has a first central longitudinal portion ZL1, a second central longitudinal portion ZL2, and a third central longitudinal portion ZL3 arranged between the first central longitudinal portion ZL1 and the second central longitudinal portion ZL2.

The first central longitudinal portion ZL1 and the second central longitudinal portion ZL2 correspond in respect of their basic structure to the central longitudinal portion ZL of Figure 1. This means that an inner tube segment 2 is provided here in each of these first and second central longitudinal portions and is embedded in the outer tube segment 3 and is surrounded completely by the outer tube segment 3. However, there is no inner tube segment arranged in the third central longitudinal portion ZL3. The third central longitudinal portion ZL3 is thus made initially only of the thermoplastic or the first polymer of the outer tube segment 3. In the exemplary embodiment of the tube assembly 1 shown in Figure 5, a ring electrode 8 is already arranged in the third central longitudinal portion ZL3 and is embedded in the outer tube segment 3. This exemplary embodiment of the tube assembly 1 is thus particularly well suited for production of an electrode, in particular an electrode intended to be implanted in a human or animal body.

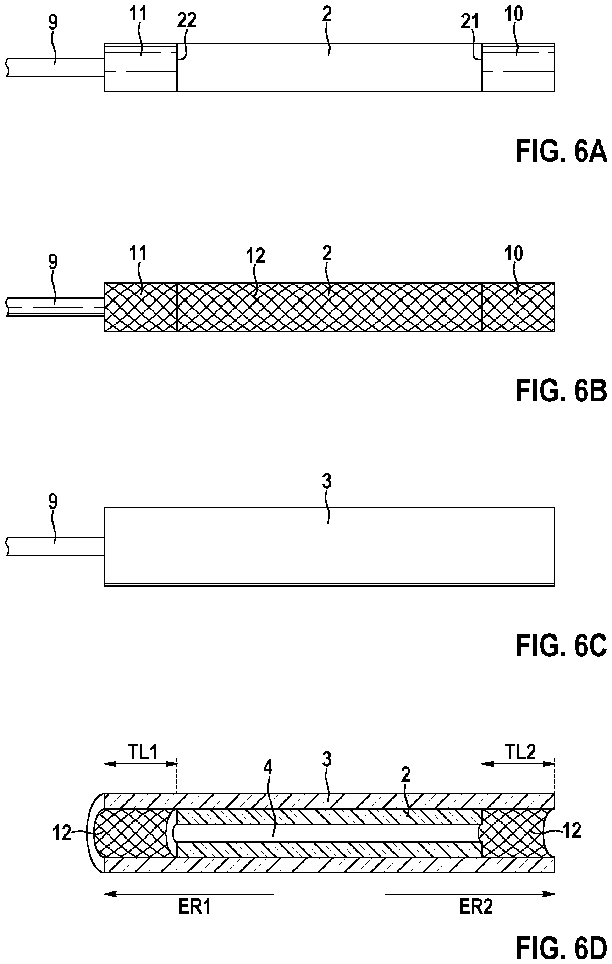

Figures 6A to 6D show different stages within the scope of a method for producing a tube assembly 1 which is equipped with a mesh.

As is shown in Figure 6A, a core wire 9 is firstly introduced into an inner lumen of the inner tube segment 2 together with a first inner sleeve 10, which is fixedly connected to the core wire 9. The first core sleeve 10 has an outer diameter corresponding to the outer diameter of the inner tube segment 2. The first core sleeve 10 is guided level with the inner tube segment 2, that is to say it contacts a first end region 21 of the inner tube segment 2.

A second core sleeve 11 is now slid over the core wire 9, more specifically until the second core sleeve 11 contacts a second end region 22 of the inner tube segment 2. The second core sleeve 11 has the same dimensions as the first core sleeve 10, that is to say its outer diameter corresponds to the outer diameter of the inner tube segment 2. If the second core sleeve 11 has contacted the second end region 22 of the inner tube segment 2, it is fixed on the core wire 9, for example by means of a clamping screw (not shown). It is then no longer possible to move the second core sleeve 11 relative to the core wire 9.

In the second method steps shown in Figure 6B, a mesh 12 is now placed around an outer side of the inner tube segment 2, the first core sleeve 10, and the second core sleeve 11. The mesh 12 is made of an aramid.

A first polymer, in particular a thermoplastic, is then extruded over the mesh 12 and thus also over the first core sleeve 10 and the second core sleeve 11, and over the inner tube segment 2. An outer tube segment 3 is thus formed. This is shown in Figure 6C.

Once the extruded polymer is solidified, the fixing between the second core sleeve 11 and the core wire 9 is broken, and the second core sleeve 11 is removed from the core wire 9 in a first removal direction ER 1. The first core sleeve 10 is then pulled from the inner lumen 4 of the inner tube segment 2 together with the core wire 9 in a second removal direction ER 2, which is opposite the first removal direction ER 1. This results in the tube assembly 1 shown in Figure 6D, comprising the inner tube segment 2, the outer tube segment 3, and the mesh 12 arranged between the inner tube segment 2 and the outer tube segment 3. The mesh 12 ensures a particularly close connection between the outer tube segment 3 and the inner tube segment 2.

Only the outer tube segment 3 together with the mesh 12, and not the inner tube segment 2, is arranged in the first terminal longitudinal portion TL1 and the second terminal longitudinal portion TL2 of the tube assembly 1. Both terminal longitudinal portions TL1 and TL2 may thus be easily connected to other components by melting the polymer of the outer tube segment 3 in these areas in order to prepare them for a welded connection.

It will be apparent to those skilled in the art that numerous modifications and variations of the described examples and embodiments are possible in light of the above teaching. The disclosed examples and embodiments may include some or all of the features disclosed herein. Therefore, it is the intent to cover all such modifications and alternate embodiments as may come within the true scope of this invention.

## Claims

1. A tube assembly having an inner tube segment (2) and an outer tube segment (3),
wherein the outer tube segment (3) completely surrounds the inner tube segment (2) in at least a first central longitudinal portion (ZL) of the inner tube segment (2) along an outer circumference of the inner tube segment (2),
wherein the outer tube segment (3) comprises a first polymer, which has a first melting range, and the inner tube segment (2) comprises a second polymer, which has a second melting range or a decomposition temperature,
wherein a mean value of the second melting range or, if the second polymer does not have a melting range, but instead only a decomposition temperature, the decomposition temperature is at least 50°C higher than a mean value of the first melting range,
**characterised in that**
the tube assembly (1) comprises a first terminal longitudinal portion (TL1), in which the outer tube segment (3) is arranged, but the inner tube segment (2) is not.

2. The tube assembly according to claim 1, **characterised in that** the tube assembly (1) comprises a second terminal longitudinal portion, in which the inner tube segment (2) is arranged, but the outer tube segment (3) is not.

3. The tube assembly according to claim 1, **characterised in that** the tube assembly comprises two terminal longitudinal portions (TL1, TL2), in which the outer tube segment (3) is arranged, but the inner tube segment (2) is not.

4. The tube assembly according to any one of the preceding claims, **characterised in that** the tube assembly (1) comprises at least one further central longitudinal portion (ZL3), in which the outer tube segment (3) is arranged, but the inner tube segment (2) is not.

5. The tube assembly according to any one of the preceding claims, **characterised in that** an inner side of the inner tube segment (2) delimits an inner lumen (4) of the tube assembly (1).

6. The tube assembly according to any one of the preceding claims, **characterised in that** the tube assembly (1) comprises a mesh (12) arranged between the inner tube segment (2) and the outer tube segment (3).

7. The tube assembly according to claim 6, **characterised in that** the mesh (12) is also arranged in the first terminal longitudinal portion (TL1) of the tube assembly.

8. The tube assembly according to any one of the preceding claims, **characterised in that** the first polymer is a thermoplastic and the second polymer is selected from the group of thermoplastics, elastomers, and thermosets.

9. The tube assembly according to any one of the preceding claims, **characterised in that** the first polymer is a polyurethane and the second polymer is a polytetrafluoroethylene or a silicone elastomer.

10. A medical product comprising a tube assembly (1) according to any one of the preceding claims.

11. The medical product according to claim 10, **characterised in that** the medical product comprises an electrode implantable in a human or animal body or a catheter insertable into a human or animal body.

12. The medical product according to claim 11, **characterised in that** the medical product is a device for stimulating the human or animal heart, a device for implanting a device for stimulating the human or animal heart, or a device for implanting a cardiac implant.

13. A method for producing a tube assembly according to any one of claims 1 to 9, said method comprising the following steps:
a) providing an inner tube segment (2), which comprises a second polymer, which has a second melting range or a decomposition temperature,
b) extruding a first polymer, which has a first melting range, over the inner tube segment in order to form an outer tube segment (3),
wherein a mean value of the second melting range or, if the second polymer does not have a melting range, but instead only a decomposition temperature, the decomposition temperature is at least 50°C higher than a mean value of the first melting range,
**characterised in that**
the first polymer is extruded also at least in a first terminal longitudinal portion (TL1) of the tube assembly (1) that is to be produced, in which the inner tube segment (2) is not arranged, such that the tube assembly (1) comprises a first terminal longitudinal portion (TL1), in which the outer tube segment (3) is arranged, but the inner tube segment (2) is not.

14. The method according to claim 13, **characterised in that,** prior to the extrusion of the first polymer, an additional tube assembly element (12, 31) is arranged ahead of or behind the inner tube segment (2) in the direction of longitudinal extent (L) or around the inner tube segment (2), wherein the first polymer then is extruded not only over the inner tube segment (2), but also over the additional tube assembly element (12, 31).

15. The method according to claim 13 or 14, **characterised in that,** prior to the step of extrusion of the first polymer, the following steps are performed:
a1) guiding a core wire (9) through an inner lumen (4) of the inner tube segment (2), wherein the core wire (9), at a first end, has a first core sleeve (10), which is fixedly connected to the core wire and which has an outer diameter greater than a diameter of the inner lumen of the inner tube segment (2), wherein the core wire (9) is guided through the inner lumen (4) until the first core sleeve (10) contacts a first end (21) of the inner tube segment (2),
a2) sliding a second core sleeve (11) onto the core wire until the second core sleeve (11) contacts a second end (22) of the inner tube segment (2), and fixing the second core sleeve (11) to the core wire (9) so that it is no longer movable relative to the core wire (9), wherein the second core sleeve (11) has an outer diameter that is greater than a diameter of the inner lumen (4) of the inner tube segment (2),
a3) applying a mesh (12) to an outer side of the inner tube segment (2), the first core sleeve (11), and the second core sleeve (11),
wherein the first polymer is then extruded over the mesh (12) and thus also over the inner tube segment (2), and then the following steps are performed:
b1) breaking the fixing of the second core sleeve (11) and removing the second core sleeve (11) from the core wire,
b2) removing the core wire (9) together with the first core sleeve (10) from the inner lumen (4) of the inner tube segment (2).
